# EUROPEAN PATENT SPECIFICATION

(11) **EP 1 566 160 B1**
(45) Date of publication and mention of the grant of the patent: **30.04.2008**
(21) Application number: 05002546.9
(22) Date of filing: 07.02.2005
(51) Int. Cl.: A61K 8/73, A61K 8/97, A61Q 11/00

(54) **Antimicrobial dental composition**
Mikrobizide Zusammensetzung für die Zähne
Composition dentaire antimicrobienne

(30) Priority: 18.02.2004 JP 2004041124
(43) Date of publication of application: 24.08.2005
(73) Proprietor: GC Corporation, Itabashi-ku Tokyo 174-8585 (JP)
(72) Inventor: Abiru, Masao GC Corporation, Tokyo (JP); Sato, Takuya GC Corporation, Tokyo (JP); Sakaguchi, Yoshihiro GC Corporation, Tokyo (JP); Sato, Kimihiko GC Corporation, Tokyo (JP)
(74) Representative: Müller-Boré & Partner Patentanwälte

(56) References cited:
- WO-A-96/02142
- US-A1- 2003 133 883
- DATABASE CA CHEMICAL ABSTRACTS SERVICE, COLUMBUS, OHIO, US; XP002342203 retrieved from STN Database accession no. 142: 100040 & KR 2001 020 067 A (SEI GOON HO) 15 March 2001 (2001-03-15)
- DATABASE CA CHEMICAL ABSTRACTS SERVICE, COLUMBUS, OHIO, US; XP002342204 retrieved from STN Database accession no. 142: 225 302 & KR 2003 047 613 A (SEI GOON HO) 18 June 2003 (2003-06-18)

## Description

The present invention relates to various antimicrobial dental compositions capable of obtaining an antimicrobial effect effectively and safely against microbes and viruses existing in an oral cavity. The antimicrobial dental compositions are, for example, dental cement, dental impression material, dentifrice or the like.

As a treatment of dental caries, the following methods are generally applied, that is, the methods comprising, directly filling a resin based material, such as a dental cement, a dental composite resin or the like, in the affected part after it is cut off, or covering the affected part with the metal from the inside of the oral cavity using a dental cement, a resin based dental adhesive or the like.

In the treatment of the dental caries, it is required that the contracted dentin is removed completely, and the same form as the original tooth is recovered after the treatment by using the dental cement so as not to cause the dental caries again after the treatment due to the microbes existing in the contacted dentin. However, since the dental caries does not uniformly progress, it is hard to completely remove the contracted dentin even if with careful attention. For this reason, an antimicrobial dental restorative material is used, and this material can asepticize the contracted dentin part still left there after removing the contacted dentin by contacting such material thereon.

As the antimicrobial dental restorative material, a material using a chemical substance having an antimicrobial activity can be used. Such chemical substance is, for example, thymol (for example, refer to Japanese Patent Laid Open No. 2000-191423), 2,4,4'-trichloro- 2'- hydroxydiphenyl ether (for example, refer to published Japanese translations of PCT international publication for patent applications No. 2001-524113), or the like. However, thymol, for example, has peculiar odor and stimulus, and other chemical substances such as chlorhexidine or the like have also peculiar odor and problems in the safety to a human body. Further, a material using a metal compound containing an ion of metal, such as, silver, copper, zinc or the like, can be used (for example, refer to Japanese Patent Laid Open No. 1989-238508). However, the metal compound has also problems in the safety to a human body and discoloration. These problems are not limited in the resin based restorative material such as dental cement, dental composite resin or the like, but also the same in the dental restorative material such as dental adhesive, sealant material, root canal filler or the like. Further, it is required for the antimicrobial dental restorative material to maintain antimicrobial effect for comparatively long time, but there is a problem that it is difficult for the above-mentioned dental compositions to maintain the effect for a comparatively long period of time.

On the other hand, when a dental prosthesis such as a denture or the like is made, the dental prosthesis is made by pressing a dental impression material to the tooth or the gum of a patient to take an impression, pouring the gypsum into the impression, hardening the gypsum to make a gypsum model, and thereby, preparing the dental prosthesis on the gypsummodel. At this time, the dental impression material directly contacts with the tooth or the gum of a patient, and the dental gypsum also contacts with the impression material, so that the dental gypsum may be polluted by the microbes or the viruses of the patient. Therefore, dentists, oral hygienists and dental technicians, who treat the dental impression material, the dental gypsum or the like, needs to pay attention carefully to virus infection, such as hepatitis, AIDS or the like, from the patient.

In a dental clinic and a dental technology laboratory, a disinfectant or an antiseptic are sprayed over the dental impression material in order to prevent infection. As the disinfectant or the antiseptic, the followings are used, that is, a glutaraldehyde preparation, a sodium hypochlorite preparation, an alcohol preparation, a povidone iodine preparation, a 2,4,4- trichloro- 2- hydroxy- diphenyl ether based preparation, or the like. However, these chemical substances have also problems with respect to the peculiar odor and the safety to a human body.

Furthermore, since microbes of many kinds live in the oral cavity other than the above-mentioned cariogenic microbes or the viruses, intraoral diseases such as periodontitis or the like caused by these microbes become a large problem. In order to suppress microbes propagation in the oral cavity, a mechanical purifying method, for example, the method comprising brushing the teeth, is widely used conventionally. This method is remarkably useful as an individual treatment, but has problems that it is difficult to learn a right brushing method and to acquire an oral hygiene custom for life.

Therefore, in order to suppress the intraoral microbes, mouth rinse, dentifrice, gel, tablets or the like are developed, where these products comprise a chemical substance having the antimicrobial activity and a solvent such as water, ethanol or the like. The chemical substance is, for example, povidone iodine, domiphen bromide, benzethonium chloride, fradiomycin sulfate, sodium azulene sulfonate, chlorhexidine, quaternary ammonium salt, or the like. However, these antimicrobial substances have also problems in the safety to a human body and the maintenance of the antimicrobial activity for a long period of time.

WO-A-96/02142 describes a deodorizing, antimicrobial and moldproofing agent which comprises chitosan, plant extracts and inorganic substances such as zeolite. Chemical Abstracts, AN 142:100040 (XP002342203); and KR-A-2001-020067 describes a toothpaste composition which is obtained by preparing an aqueous cyclodextrin solution, adding an essential oil mixture such as basil, bergamot, chamomile, cedarwood, clary sage, eucalyptus, lavender, rosemary, mint, geranium, juniper, cinnamon, jasmine, grape fruits, rose wood or marjoram. US-A-2003/133883 describes oral care compositions including grapefruit seed extract in synergistic combination with an ion-providing compound. Chemical Abstracts, AN 142:225302 (XP002342204); and KR-A-2003-047613 describes a toothpaste composition containing an antimicrobial neem tree extract together with cyclodextrin.

In order to solve the above-mentioned conventional problems of the antimicrobial dental composition, the present inventors noted that some kinds of materials extracted from a natural product have an excellent antimicrobial activity, from the record on the past experience. Such a material extracted from the natural product is, for example, an extract of green tea or oolong tea, a grapefruit seed extract, a leaf of rosemary or cyclobalanopsis salicina, mustards, a bamboo extract, a ume vinegar or the like. Further, the present inventors found out that the grapefruit seed extract and a persimmon juice extract among these materials are excellent from the points of effect and safety. However, the grapefruit seed extract and the persimmon juice extract are liquid using water, ethanol or the like, so that these extracts are inconvenient to treat. Further, these extracts are hardly applied for giving the antimicrobial property to the dental composition which needs to take a form of a paste or a tablet. In addition, there is a problem in the maintenance of the antimicrobial activity for a long period of time.

The primary object of the present invention is to provide an antimicrobial dental composition which has high safety without stimulus and antimicrobial activity for a long period of time. Further, the primary object thereof is to provide the antimicrobial dental composition which is advantageous to a paste or solid-like dental composition.

The earnest work was carried out in order to solve the above-mentioned problems and, as the result, the present inventors noted that the grapefruit seed extract and the persimmon juice extract could be contained in the dental composition as a powder where these extracts were included in cyclodextrin, in order to maintain the antimicrobial activity for a long period of time and obtain the paste or solid-like antimicrobial dental composition. Thus, the antimicrobial dental composition according to the present invention was completed.

That is, the present invention is the antimicrobial dental composition, which contains a powder containing a grapefruit seed extract and a persimmon juice extract, where these extracts are included in cyclodextrin, or which contains a powder containing a grapefruit seed extract included in cyclodextrin and a powder containing a persimmon juice extract included in cyclodextrin, wherein the blending ratio of the persimmon juice extract / the grapefruit seed extract being 3 / 7 to 7 / 3 by weight.

It is preferable that the antimicrobial dental composition is used as a dental restorative material, a dental restoration auxiliary material or an oral hygiene material.

The antimicrobial dental composition according to the present invention has high safety without stimulus and has an antimicrobial activity for a long period of time, and further, is advantageous for the paste or solid-like dental composition.

In the present invention, the grapefruit seed extract is obtained by extracting from a seed of a grapefruit (Citrus paradisi MACF.), and the persimmon juice extract is obtained by extracting from a fruit of a persimmon (Diospyros kaki Thunberg Ebenaceae), and these extracts are extracted with water or ethanol.

Both of the main components of these extracts are fatty acid and flavonoid. The grapefruit seed extract and the persimmon juice extract have excellent antimicrobial effect and high safety to a human body respectively. The grapefruit seed extract is useful to cariogenic microbes such as general microbes, fungus, Pseudomonas aeruginosa, Streptococcus or the like. The main component of the persimmon juice extract is condensation catechin. The condensation catechin has the excellent antimicrobial activity with respect to Gram-positive bacteria, Gram-negative bacteria, fungus or the like. It is considered that the main reason of this efficacy is that the condensation catechin has a phenol effect of flavonoids and an electrostastic effect of fatty acid. Further, it can be expected that both of these extracts have an excellent action to suppress proliferation to Streptococcus mutans.

When the microbes existing in the dental plaque can not be fully killed only by the antimicrobial force of the grapefruit seed extract, the simultaneous use of these extracts is effective. That is, when these extracts are simultaneously used, a dental plaque decomposing activity can be also simultaneously advanced since the catechin in the persimmon juice extract is quickly combined with protein in the dental plaque as compared with other catechins, and thus there is an advantage that the antimicrobial effect can be expected within the dental plaque.

As a making method of the powder in which the grapefruit seed extract and the persimmon juice extract are included in cyclodextrin, a conventional method can be used. A general method comprises, mixing the grapefruit seed extract and the persimmon juice extract with a cyclodextrin- containing liquid, and drying the containing liquid by a freeze-dry, spraying or the like.

As cyclodextrin used in the present invention, the cyclodextrin used in a conventional inclusion technique can be used, for example, a following cyclodextrin derivative can be used, that is, α-cyclodextrin, β- cyclodextrin, γ- cyclodextrin, or a branched cyclodextrin derivative in which 1to 4 glucose molecules are α-1-6 linked as a branch to these cyclodextrins.

The antimicrobial dental composition according to the present invention can be applied to various dental compositions used in a conventional dentistry. More particularly, the composition is useful as a dental restorative material, a dental restoration auxiliary material, or an oral hygiene material for prevention such as a dentifrice or the like.

More particularly, as the dental restorative material, the following direct restorative materials can be mentioned. That is, the material comprising, a denture base material, such as a denture base resin, a resin for a relining material for denture, a resin for expanding denture, an orthodontic resin for observing a sprint or a facet or the like, a denture stabilizer, a dental cement or a dental resin material for luting, bonding, filling, lining and temporary sealing, a mixed material of a dental cement for luting, bonding, filling, lining and temporary sealing and a dental resin material for luting, bonding, filling, lining and temporary sealing, a root canal filler such as gutta-percha point for filling root canal or the like. These dental restorative materials can be directly used in the oral cavity and used for a comparatively long period of time.

Further, as the dental restoration auxiliary material, the following indirect restorative materials can be mentioned. That is, the material comprising, an alginate impression material, a rubber based precision impression material such as a silicon impression material or the like, an agar impression material, a modeling compact, a mouth protector, various denture base compatible diagnosing materials, a base excessive pressure diagnosing material, an adhesive for an impression material, a dental gypsum, a dental refractory investment, or the like. These materials are used for making a diagnostic material or a dental prosthesis in the oral cavity, which contacts with the inside of the oral cavity temporarily or for a comparatively short period of time.

Furthermore, as the dental composition, the followings can be mentioned other than the above-mentioned various dental materials, that is, a dentifrice showing a form of a paste, a gel, a troche, a tablet or the like and an oral hygiene material relating to an oral cavity cleaning, such as a mouth rinse, a dental caries preventing material or the like.

The antimicrobial dental composition according to the present invention can be prepared most easily by blending with the conventional dental composition mentioned above. In the case of the dental cement, the alginate impression material powder or the denture stabilizing material, which comprise a powder material and water, it is preferable that extract powders, in which the grapefruit seed extract and/or the persimmon juice extract are included in cyclodextrin, are blended 0.01-10 wt.% in the powder material. If the blending amount is less than 0.01 wt.%, the antimicrobial effect is hardly obtained, and if the blending amount exceeds 10 wt.%, the function of the main dental composition may be lost.

When the dental composition is a solid, such as the paste, the gel, the troche, the tablet or the like, it is preferable that the extract powder, in which the grapefruit seed extract and the persimmon juice extract are included in cyclodextrin, are blended 0.01-40 wt.%, preferably 0.1-20 wt.% to the whole of the solid composition. If the blending amount is less than 0.01 wt.%, the antimicrobial effect is hardly obtained, and if the blending amount exceeds 40 wt.%, the function of the main dental composition may be lost.

As the grapefruit seed extract and the persimmon juice extract used in the present invention, Desfan-100 (product name) imported by Mitsuba Trading Co., Ltd., can be used as the grapefruit seed extract, and Pancil BA (product name) produced by Rilis Science Co., Ltd. , can be used as the persimmon juice extract. According to the present invention, the grapefruit seed extract and the persimmon juice extract are blended simultaneously, and the following two ways are considered. One is that these extracts are mixed at first and included in cyclodextrin to make the powder. In this case, it is preferable that the blending ratio of the persimmon juice extract / the grape fruit extract is 3 / 7 to 7 / 3 by weight. The other is that each extract is included in cyclodextrin respectively at first and these included powders are mixed to be used. In this case, it is preferable that the blending ratio of the included persimmon juice extract powder / the included grape fruit extract powder is 3 / 7 to 7 / 3 by weight. In the case that the microbes existing in an inside of the dental plaque cannot be fully killed only by the antimicrobial force of the grapefruit seed extract, if these extracts are blended in such ratio, the dental plaque decomposing activity can advance using the characteristic that the catechin of the persimmon juice extract is quickly combined with protein of the dental plaque as compared with other catechins, and thus the antimicrobial effect can be expected with respect to the inside of the dental plaque.

### [Example]

Hereinafter, the present invention is explained specifically with examples, but it is not limited to these examples.

### <Powder contains the grapefruit seed extract and the persimmon juice extract included in cyclodextrin (Powder A)>

The powder A is prepared by mixing 40 wt.% commercially available cyclodextrin (IsoElite P (product name) produced by ENSUIKO Sugar Refining Co., Ltd.), 20 wt.% grapefruit seed extract (Desfan-100 (product name) produced by Mitsuba Trading Co., Ltd.), 20 wt.% persimmon juice extract (Pancil BA (product name) produced by Rilis Science Co., Ltd.) and 20 wt. % water, and powdered these by freeze-drying. The commercially available cyclodextrin has branched α-cyclodextrin, branched β- cyclodextrin and branched γ- cyclodextrin as the main component.

### <Powder containing the grapefruit seed extract included in cyclodextrin (Powder B)>

The powder B is prepared by mixing 30 wt.% cyclodextrin (K-100 (product name) produced by ENSUIKO Sugar Refining Co., Ltd.), 20 wt.% grapefruit seed extract (Desfan-100 (product name) produced by Mitsuba Trading Co., Ltd.) and 50 wt.% water, and powdering these by spray-drying. The commercially available cyclodextrin has α- cyclodextrin as the main component.

### < Powder containing the persimmon juice extract included in cyclodextrin (Powder C)>

The powder C is prepared by mixing 30 wt.% cyclodextrin (K-100 (product name) produced by ENSUIKO Sugar Refining Co., Ltd.), 20 wt.% persimmon juice extract (Pancil BA (product name) produced by Rilis Science Co., Ltd.) and 50 wt.% water, and powdering it by freeze drying. The commercially available cyclodextrin has α- cyclodextrin as the main component.

### Example 1

An antimicrobial dental cement was prepared by mixing 2 wt.% powder A to a commercially available glass ionomer cement powder (Fuji I Powder (product name) produced by GC Corporation) and fully stirring it. A special liquid (Fuji I Liquid (product name) produced by GC Corporation) was added with prescribed amount to the antimicrobial dental cement to be kneaded. The kneaded liquid was poured into a cylindrical mold of 6mm in diameter × 6mm in height to be hardened. A test sample was taken out of the mold to be used to an antimicrobial test mentioned below.

### Example 2

An antimicrobial dental impression material was prepared by mixing 7 wt.% powder A to the powder of a commercially available alginate dental impression material (AROMA FINE DF III (product name) produced by GC Corporation), and fully stirred. Water was added in prescribed amount to the powder of dental impression material to be kneaded. The mixture was taken into the cylindrical mold of 6mm in diameter × 6mm in height to be gelled. A test sample was taken out of the mold to be used to an antimicrobial test mentioned below.

### Example 3

An antimicrobial resin for a relining material for denture was prepared by mixing 6 wt.% powder A to a commercially available resin for a lining material for denture (MILD REBARON Powder (product name) produced by GC Corporation), and fully stirred. A special liquid (MILD REBARON Liquid (product name) produced by GC Corporation) was added in prescribed amount to the antimicrobial resin to be kneaded. The mixture was taken into the cylindrical mold of 6mm in diameter × 6mm in height to be hardened. A test sample was taken out of the mold to be used to an antimicrobial test mentioned below.

### Example 4

The following materials were mixed to prepare a first paste.
That is,
35 wt.% polyacrylic acid having weight average molecular weight 20,000,
37 wt.% distilled water,
27 wt. % silane-treating quartz powder, and
1 wt.% camphorquinone,
where the silane-treating quartz powder was prepared by adding 20g of an ethyl alcohol solution of 10% γ-methacryloxypropyltrimethoxysilane to 100g of a fine quartz powder having an average particle diameter 10 µm, fully stirred in a mortar, and dried at 110°C for 2 hours with a dryer.

The following materials were mixed to prepare a second paste. That is,
61 wt.% commercially available glass ionomer cement (Fuji I Powder (product name) produced by GC Corporation),
8 wt.% hydroxyethylmethacrylate,
8wt.%2-hydroxy, 1- acryloxy, 3- methacryloxy propane, 17 wt.% Di- 2- methacryloxyethyl- 2,2,4- triethyl hexamethylene dicarbamate,
2 wt.% glycidyl methacrylate,
2 wt.% powder B, and
2 wt.% powder C.

The first paste and the second paste were mixed to prepare an antimicrobial paste-like dental glass ionomer cement.

0.5g of the first paste and 2.5g of the second paste were kneaded for 10 seconds by using a mixing pad and a spatula. The kneaded pastes were taken into the cylindrical mold of 6mm in diameter × 6mm in height, and irradiated from both sides for 60 seconds by a visible ray irradiator (NEWLIGHT VL-II (product name) produced by GC Corporation) to be hardened as in the case of the conventional glass ionomer cement. A test sample was taken out of the mold to be used to an antimicrobial test mentioned below.

### Example 5 (Reference Example)

An antimicrobial dental root canal filling material was prepared by weighing following each component and fully kneading these components with a kneader.
That is,
5 wt.% trans-polyisoprene,
0.1 wt.% ethylene-vinyl acetate copolymer resin (a softening point: 60°C),
93.8 wt.% zinc chloride and
1.1 wt.% powder B :

This composition was softened at 130°C, and taken into the cylindrical mold of 6mm in diameter × 6mm in height, and upper and lower sides thereof were pressed by a metallic plate, and cooled to be formed. A test sample was taken out of the mold to be used in an antimicrobial test mentioned below.

### Example 6

An antimicrobial dental gypsum was prepared, mixing 3 wt. % powder B and 2 wt.% powder C to a powder of a commercial dental gypsum (NEW PLASTONE (product name) produced by GC Corporation) and fully stirred. Water was added in prescribed amount to the gypsum to be kneaded and the mixture was taken into the cylindrical mold of 6mm in diameter × 6mm in height to be hardened. A test sample was taken out of the mold to be used to an antimicrobial test mentioned below.

### Example 7

An antimicrobial dentifrice was prepred, weighing each following component and fully kneading.
That is,
40 wt.% calcium carbonate,
1.5 wt.% sodium lauryl sulfate,
1.0 wt.% carboxymethylcellulose sodium,
8 wt.% glycerol,
29.3 wt.% polyethylene glycol,
20 wt.% sorbitol, and
0.2 wt.% powder A.

This dentifrice was filled in the cylindrical mold of 6mm in diameter × 6mm in height, and a test sample was used in an antimicrobial test mentioned below without taking it out of the mold.

### Example 8 and Reference Example 9

Following each component was weighed to be mixed.
That is,
32 wt.% lactose,
10 wt.% carboxymethylcellulose sodium,
26 wt.% crystalline cellulose,
8 wt.% polyethylene glycol,
4 wt.% magnesium stearate,
18 wt.% xylitol, and
2 wt.% Powder A (or Powder B).

10g of the above mixed components were molded with 0.5kg hardness by using a single-loader tableting apparatus (2B Type (product name) produced by KIKUSUI SEISAKUSYO LTD.) to prepare a tablet. Furthermore, a tablet-like composition for an oral cavity was prepared as an antimicrobial tablet in an oral cavity, by aging the tablet at 40°C and 75% relative humidity for 12 hours. A tablet-like composition using the powder B instead of the powder A was made as Reference Example 9. Removabilities of microbes in an oral cavity of these compositions were evaluated by a test mentioned below.

### Examples 10 and Reference Example 11

Following each component was weighed to be mixed.
That is,
35 wt.% mannitol,
10 wt.% carboxymethylcellulose calcium,
8 wt.% polyethylene glycol,
2 wt.% sugar ester,
18 wt.% xylitol,
15 wt.% sodium hydrogencarbonate,
10 wt.% Tartaric acid, and
2 wt.% Powder A (or Powder C).

10g of the above mixed components were molded with 0.5kg hardness by using a single-loader tableting apparatus (2B Type (product name) produced by KIKUSUI SEISAKUSYO LTD.) to prepare a tablet-like composition for an oral cavity as an antimicrobial tablet in an oral cavity having the antimicrobial activity. A tablet-like composition using the powder C instead of the powder A was prepared as Reference Example 11. Removabilities of microbes in an oral cavity of these compositions were evaluated by a test mentioned below.

### Comparison Examples

Compositions not containing the extract powders in Examples 1-3 and 6 were prepared as Comparison Examples 1-3 and 6 respectively, where the grapefruit seed extract and the persimmon juice extract were included in cyclodextrin in the extract powders. Comparison Example 4 relates to the composition in Example 4 where the powders B and C were replaced with glycidyl methacrylate. Comparison Example 5 relates to the composition in Example 5 where the powder B was replaced with zinc oxide. Comparison Example 7 relates to a composition of Example 7 where the powder A was replaced with sorbitol. Comparison Examples 8-11 relate to compositions in Examples 8-11 where the powders A to C were replaced with xylitols.

### < An antimicrobial property test>

Antimicrobial property tests of dental compositions described in Examples 1-7 and Comparison Examples 1-7 were carried out with following processes.

100 µL Glycerol Stocks of S. Mutans ATCC25175 Stock and S. Sobrinus ATCC33475 Stock were inoculated in about 20mL BHI liquid mediums respectively, and cultured by standing overnight at 37°C. About 2 × 10⁷ cells/mL of S. Mutans ATCC25175 and S. Sobrinus ATCC33475 suspension were prepared from this preculture solution, and 100µL of these suspensions were smeared to a MSB agar medium. On the mediums smeared with the suspensions of microbes, two pieces of the antimicrobial dental composition (test samples) of Example 1 were put respectively. Then, two pieces of composition of Comparison Example 1 were similarly put on the MBS agar mediums smeared with the suspensions of microbes held with filter papers. Examples 2-7 and Comparison Examples 2-7 were tested similarly.

Each set was cultured at 37°C for 2 days, and sizes of formed inhibition circles (larger ones of two of each piece) were compared. As for the antimicrobial properties of each example and comparison example, the trend of the antimicrobial activity was evaluated as follows from the size of the inhibition circle, and these results were shown collectively in Table 1.

A: The inhibition circle of the example was larger than that of the comparison example, and the antimicrobial effect was high.

B: The inhibition circle of the example was a little larger than that of the comparison example, and it was considered that there was the antimicrobial effect.

C: There was almost no inhibition circle, and the antimicrobial effect was not expected.

**[Table 1]**

| | 1 | 2 | 3 | 4 | 5 | 6 | 7 |
|---|---|---|---|---|---|---|---|
| Examples | B | A | A | A | B | A | B |
| Comparison Examples | C | C | C | C | C | C | C |

Antimicrobial property tests of dental compositions described in Examples 8-11 and Comparison Examples 8-11 were carried out with following processes.

The number of mutans Streptococcus in a saliva was measured using MSB (mitis-salivarius bacitracin medium). The saliva extracted from a testee was diluted using PBS under the aseptic conditions. 50 µL of the diluted saliva was smeared to the medium, and cultured under the anaerobic conditions at 37°C for 2 days. Then, the quantity of mutans Streptococcus before inspection (CFU/mL) was calculated by measuring the number of colonies.

The saliva of the testee was extracted immediately after biting the tablet of Example 8, and the number of mutans Streptococcus in this saliva was calculated by the same process as the above-mentioned one. The ratio (%) of the number of mutans Streptococcus of the saliva after biting and before biting was shown in Table 2. In this case, the ratio of the numbers of mutans Streptococcus before and after the test in Examples 9-11, Comparative Examples 8-11 was obtained similarly to the above mentioned process with enough time interval.

**[Table 2]**

| | 8 | 9 | 10 | 11 |
|---|---|---|---|---|
| Examples | 50.3% | 63.7% | 49.5% | 58.4% |
| Comparison Examples | 99.4% | 99.6% | 111.5% | 103.7% |

The antimicrobial effects of the antimicrobial dental compositions of Examples 1-7 can be confirmed from Table 1. However, the antimicrobial effects of the antimicrobial dental compositions of Comparison Examples 1-7 cannot be confirmed. Further, the antimicrobial effects of the antimicrobial dental compositions of Examples 8-11 can be confirmed from Table 2. However, the antimicrobial effects of the antimicrobial dental compositions of Comparison Examples 8-11 cannot be confirmed.

Therefore, it can be confirmed that the antimicrobial dental composition according to the present invention has the antimicrobial effect, and has high value to contribute to the dentistry field.

## Claims

1. An antimicrobial dental composition which contains a powder containing a grapefruit seed extract and a persimmon juice extract, where these extracts are included in cyclodextrin, or which contains a powder containing a grapefruit seed extract included in cyclodextrin and a powder containing a persimmon juice extract included in cyclodextrin, wherein the blending ratio of the persimmon juice extract / the grapefruit seed extract being 3 / 7 to 7 / 3 by weight.

2. The antimicrobial dental composition according to Claim 1, wherein said composition is a dental restorative material.

3. The antimicrobial dental composition according to Claim 1, wherein said composition is a dental restoration auxiliary material.

4. The antimicrobial dental composition according to Claim 1, wherein said composition is an oral hygiene material.

5. The antimicrobial dental composition according to any one of Claim 1-4, wherein when said composition comprises a powder material and an aqueous solution, the powder containing the grapefruit seed extract and/or the persimmon juice extract being included in cyclodextrin is blended 0.01-10 wt.% in said powder material.

6. The antimicrobial dental composition according to any one of Claim 1-4, wherein when said composition is a solid, such as a paste, a gel, a troche or a tablet, the powder containing the grapefruit seed extract and/or the persimmon juice extract being included in cyclodextrin is blended 0.01-40 wt.% in said solid.

## Patentansprüche

1. Antimikrobielle Dentalzusammensetzung,
welche ein Pulver enthält, enthaltend ein Grapefruitsamenextrakt und ein Persimon- bzw. Dattelpflaumensaftextrakt, wobei diese Extrakte in Cyclodextrin eingeschlossen sind, oder
welche ein Pulver enthält, enthaltend ein Grapefruitsamenextrakt, eingeschlossen in Cyclodextrin, und ein Pulver, enthaltend ein Persimonsaftextrakt, eingeschlossen in Cyclodextrin,
wobei das Mischungsverhältnis von Persimonsaftextrakt / Grapefruitsamenextrakt von 3 / 7 bis 7 / 3, bezogen auf das Gewicht, beträgt.

2. Antimikrobielle Dentalzusammensetzung nach Anspruch 1, wobei die Zusammensetzung ein Dentalwiederherstellungsmaterial ist.

3. Antimikrobielle Dentalzusammensetzung nach Anspruch 1, wobei die Zusammensetzung ein Dentalwiederherstellungshilfsmaterial ist.

4. Antimikrobielle Dentalzusammensetzung nach Anspruch 1, wobei die Zusammensetzung ein Mundhygienematerial ist.

5. Antimikrobielle Dentalzusammensetzung nach einem der Ansprüche 1 bis 4,
wobei, wenn die Zusammensetzung ein Pulvermaterial und eine wässerige Lösung umfasst, das Pulver, enthaltend das Grapefruitsamenextrakt und/oder das Persimonsaftextrakt, eingeschlossen in Cyclodextrin, zu 0,01 bis 10 Gew.-% in das Pulvermaterial gemischt ist.

6. Antimikrobielle Dentalzusammensetzung nach einem der Ansprüche 1 bis 4,
wobei, wenn die Zusammensetzung ein Feststoff ist, wie eine Paste, ein Gel, eine Pastille oder eine Tablette, das Pulver, enthaltend das Grapefruitsamenextrakt und/oder das Persimonsaftextrakt, eingeschlossen in Cyclodextrin, zu 0,01 bis 40 Gew.-% in den Feststoff gemischt ist.

## Revendications

1. Composition dentaire antimicrobienne qui contient une poudre contenant un extrait de pépins de pamplemousse et un, extrait de jus de kaki, où ces extraits sont inclus dans une cyclodextrine, ou qui contient une poudre contenant un extrait de pépins de pamplemousse inclus dans une cyclodextrine et une poudre contenant un extrait de jus de kaki inclus dans une cyclodextrine, dans laquelle le rapport de mélange de l'extrait de jus de kaki/l'extrait de pépins de pamplemousse est de 3/7 à 7/3 en poids.

2. Composition dentaire antimicrobienne selon la revendication 1, dans laquelle ladite composition est un matériau de restauration dentaire.

3. Composition dentaire antimicrobienne selon la revendication 1, dans laquelle ladite composition est un adjuvant de restauration dentaire.

4. Composition dentaire antimicrobienne selon la revendication 1, dans laquelle ladite composition est une substance pour l'hygiène buccale.

5. Composition dentaire antimicrobienne selon l'une quelconque des revendications 1 à 4, dans laquelle ladite composition comprend un matériau de poudre et une solution aqueuse, la poudre contenant l'extrait de pépins de pamplemousse et/ou l'extrait de jus de kaki étant inclus dans une cyclodextrine est mélangée à 0,01 à 10 % en poids dans ledit matériau de poudre.

6. Composition dentaire antimicrobienne selon l'une quelconque des revendications 1 à 4, dans laquelle ladite composition est un solide, tel qu'une pâte, un gel, une pastille ou un comprimé, la poudre contenant l'extrait de pépins de pamplemousse et/ou l'extrait de jus de kaki étant inclus dans une cyclodextrine est mélangée à 0,01 à 40 % en poids dans ledit solide.
